# EUROPEAN PATENT SPECIFICATION

(11) **EP 1 454 623 B1**
(45) Date of publication and mention of the grant of the patent: **30.06.2010**
(21) Application number: 04004805.0
(22) Date of filing: 02.03.2004
(51) Int. Cl.: A61K 9/70, A61L 15/00, A61F 13/00

(54) **Pad with a gel layer having cosmetic or therapeutic activity**
Gelbeschichtete Einlage mit kosmetischer oder therapeutischer Anwendung
Compresse pourvue d'une couche de gel à utilisation cosmétique ou thérapeutique

(30) Priority: 06.03.2003 IT MI20030414
(43) Date of publication of application: 08.09.2004
(73) Proprietor: BIOFARMITALIA S.p.A., 20144 Milano (IT)
(72) Inventor: Pinna, Fausto, 20050 Lesmo (M) (IT); Pinna, Marco, 21056 Induno Olona (VA) (IT)
(74) Representative: Frignoli, Luigi

(56) References cited:
- EP-A- 0 303 445
- EP-A- 0 392 845
- EP-A- 1 170 020
- WO-A-02/072081
- US-A- 5 547 681

## Description

The present invention relates to a breathable pad, patch or the like, a surface of which is at least partially covered by a layer of semi-solid gel able to develop a decongestant, cosmetic or therapeutic action.

Physical or physiological states or alterations of the human organism very frequently determine conditions which must be counteracted using various remedies. For example, in the case of a simple cold, balsamic inhalations are used (in the form of nasal sprays, vapours, aerosols and the like) in order to free the airways of the nose; creams and ointments are used to counteract reddening of the lower part of the nose and the upper lip (due mainly to the rubbing of the handkerchief used to blow the nose); and cold water or ice packs are used to alleviate the sensation of heat.

Another example, in the case of ocular or periocular traumas or in the period immediately following eye surgery, the affected area is cooled with ice or water to prevent or reduce localized swelling (by developing a localized cryogenic action); active substances with anti-bacterial, anti-edema, decongestant and analgesic effect are applied to the affected area (to develop a therapeutic action); and the eye is covered with a pad, to impart an occlusive effect to the eye to retain on the eye the soothing, balsamic, aromatic and anti-bacterial vapours which have evolved from substances applied to the ocular region before the pad was rested onto it.

In these and in a large number of other cases it is therefore necessary to carry out a succession of separate operations which not only require time and attention but can often be carried out incorrectly.

Transdermal patches or pads having a drug reservoir layer on a backing sheet are known in the art. EP-A-0303445 discloses a patch on which a gel base contains clebopride, a water-soluble polymer, water and a water-holding agent, the use of such a patch permitting adequate concentrations of clebopride to be maintained over prolonged periods of time.

EP-A-1170020 discloses an external skin patch having a coating of a drug-containing base on a substrate, the base comprising a gel containing a soluble polymeric material , a cross-linking agent, a local anestetic and an analgesic agent as medicinal components; such patch has an excellent drug release controlling function and allows the drug to be transdermally absorbed for an extended period of time.

WO02/072081A deals with a topical patch comprising a support on one surface of which there is a gel, water and a delayed-type hypersensitivity inducer. When used, the topical patch preparations are applied to a skin surface of a subject and maintained on application for a period of time sufficient for an effective amount of the inducer to be administered to the subject through its skin.

US 5,547,681A shows dermal patches which are based on crosslinked polymeric gel comprising polyvinyl alcohol and a crosslinking agent and comprising a multivalent transition element (e.g. titanium derivatives). Such patches have a high degree of water absorbency and oxygen permeability which makes them suitable to carry and deliver a pharmaceutical active agent to the dermal surface of a mammal, but they can also be used without any pharmaceutically active agent at all, which renders them suitable for treatment of open skin, e.g. lacerations, burns or blisters.

EP-A-0392845 also discloses a topical and transdermal delivery system consisting of a solid gel based on polyvinyl alcohol or other hydrogels (of the water and a drug in suspension or in emulsion therewith. The gel can be applied as such on the skin or it an be coated on a support member such as a woven or unwoven fabric). The user may apply the creamy gel to the desired location and then he sprays, paints or otherwise applies a gelling agent to the cream which solidifies on the side that does not contact the skin, forming a vapour barrier preventing evaporation of water and curbs the loss of volatile active ingredients (see, for example, the passage bridging pages 6 and 7 of the description; page 7, lines 20-22; page 8, lines 9-25; Examples 4-6; page 12, lines 29-34). Alternatively, the cream can be used with the gelling means-impregnated absorbent substrate or as a pre-formed gel provided on a substrate (page,12, lines 21-23).

In any case, the gelling agents or means form a vapour barrier as summarized hereabove. As a consequence, these patches provide a slightly warming feel to the subject to which they are applied for a period in excess of 4 hours each (see page 11, lines 8-14).

The main object of the present invention is to form a flexible pad, one surface of which carries a layer of gel, and which can be applied to the human skin for use in developping a decongestant, cosmetic and/or pharmaceutical action.

These objects are attained by a pad consisting of a flexible and porous support, on at least one surface of which a layer of gel is applied consisting of an intimate mixture comprising between 50% and 77% of water, between 6.5% and 44% of polyvinyl alcohol, between 0% and 10% of a substance of plant origin comprising essential oils and aromatic extracts; between 0% and 10% of at least one dermatologically compatible component chosen from the group comprising soothing, skin repairing, cicatrising, anti-inflammatory, antiseptic and bactericidal substances; said gel further comprising between 0.01 % and 5.2% of an alkaline or alkaline earth metal tetraborate, the percentages being by weight.

Preferably said support consists of a non-woven fabric made with fibres chosen from the group comprising viscose, polyester and polyethylene fibres, mixed polyester/viscose fibres, cotton and linen, said fabric support having a thickness between 15 and 200 microns, a density from 65 to 145 g/dm³ and a weight between 15 and 200 g/m².

If required, said flexible porous support can be impregnated by the aforementioned substance of plant origin with a quantity up to 10% of the weight of the gel layer applied to the flexible support itself.

The gel can also contain between 0.001% and 5.2% by weight of at least one dermatologically compatible auxiliary component chosen from the group consisting of solvents, wetting agents, preservatives, emulsifiers, stabilizers, solubilizers, surfactants and colorants.

Preferably, in the gel the water is present in a quantity of about 71 %, the polyvinyl alcohol of about 25%, substances of plant origin of about 5%, soothing, skin repairing, cicatrising, anti-inflammatory, antiseptic or bactericidal substances of about 5%, the percentages being by weight.

The preferred essential oils and aromatic extracts are chosen from oils and extracts of silver fir, star anice, bitter orange, sweet orange, benzoin absolute, bergamot, cajeput, Roman chamomile, white camphor, cinnamon leaves, carrot seeds, citron fruits, cypress, citronella, eucalyptus globulus, extra sweet fennel, cloves, jasmine flowers, bourbon geranium, juniper berries, triple distilled juniper, incense absolute, iris, true lavender, cedarwood, guaiacan wood, rosewood, lemongrass, deterpenated lemon, lemon extra, litsea cubeba, marjoram, mandarin, Melissa, triple distilled peppermint, peppermint, mimosa, myrrh, myrtle, neroli oil, niaouli, palmarosa, patchouli, petitgrain, Scots pine, Bulgarian rose, Riviera rose, rosemary, deterpenated rosemary, clary sage, Mysore sandalwood, storax absolute, white thyme, vanilla, verbena, vetiver, ylang-ylang, ginger, menthol, tea tree.

Due to the warmth of the human skin to which the gel, spread onto the patch, is applied, the water begins to evaporate from the free surface of the gel, so causing cooling (cryogenic effect) of that part of the skin on which the gel is applied. Evaporation of the water causes molecules of aromatic substances (for example essential oils) which are possibly present in the gel to be drawn outwards, with consequent controlled release of aromatic and/or balsamic vapours which can be retained in contact with the skin for a long time since the gel is spread on a support, pad or patch applied to the skin itself. By osmotic effect, part of the water present in the gel flows into the epidermal interstices (with a consequent hydrating effect on the skin itself) favouring the penetration and absorption into the skin of any cosmetic or pharmaceutical substances possibly dispersed within the gel.

As the gel is spread onto a flexible porous support (pad or patch or the like), such porosity determines the rate (i.e. the duration in time) of water evaporation, thus enabling control of the rate or duration of transfer of cosmetic and pharmacological substances contained in the gel from the gel to the skin.

The possible application of a film made of plastic material, placed between the flexible support and the gel layer, can drastically reduce and even completely eliminate evaporation of water from the skin to promote its hydration, giving a physiological environment with a high level of humidity.

Some embodiments will now be described to further clarify the understanding of the nature, shape and structure and of the method of obtaining the pad.

### EXAMPLE 1

### Preparation of a decongestant nasal patch

30 kg of demineralised water, 0.120 kg of parabens (preserving agents) and 5.6 kg of polyvinyl alcohol are fed into a continuous mixer, heated to 70°C; mixing is continued until a uniform mass (Phase A) is obtained in which the polyvinyl alcohol is completely dissolved in the water, the mass then being cooled to ambient temperature.

Separately, 1.2 kg of demineralised water and 0.300 kg of sodium tetraborate are fed into a steel mixer heated to a temperature between 25° and 30°C, and stirred until dissolution is complete to give a uniform mass (Phase B).

0.180 kg of monopropylene glycol, 0.140 kg of 70% sorbitol, 5 kg of a mixture of extracts of Aloe Vera, Calendula and Hypericum perforatum, 3 kg of Eucalyptus, mint and white thyme essential oils and 20 kg of sodium alginate are added at ambient temperature (Phase C) to the same kneader (mixer) in which Phase A was prepared: the mass obtained in this manner is maintained under agitation for about 15-20 minutes while reversing the mixer rotation direction several times. The aforesaid Phase B is slowly added as a thin stream to this mass and mixing is continued for about 20-30 minutes to thus form a gel in which the components of plant origin are intimately dispersed. The fluidity of the gel can be increased by increasing the amount of Phase A added to the mixer; vice-versa, the fluidity can be decreased by adding further quantities of Phase B to the mixer.

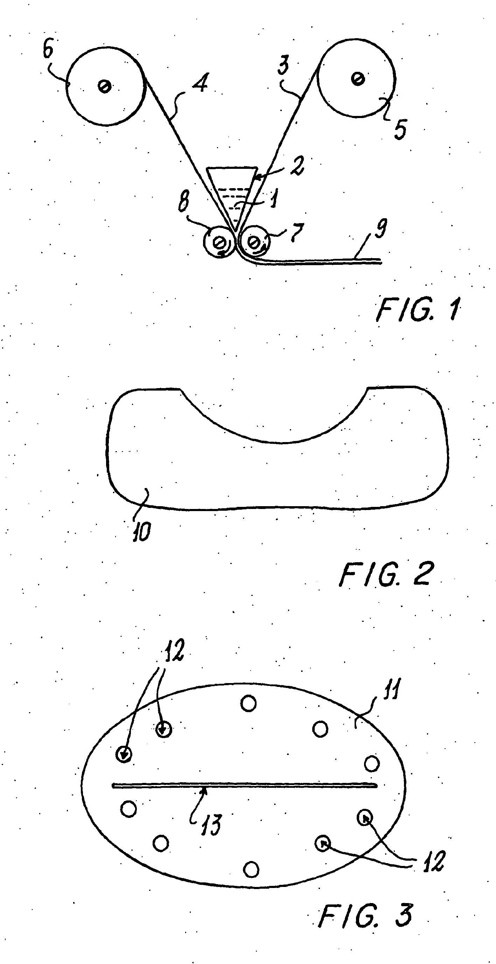

The gel thus obtained has cryogenic, cosmetic (balsamic) and cicatrising characteristics. A mass of said gel 1 is placed in a doctor blade 2 (Figure 1) and is spread between two continuous bands 3 and 4 originating from two bobbins 5 and, respectively, 6.

The band 3 consists of a non-woven fabric of viscose fibres (65%) and polyester fibres (35%) forming a web (of the type known as "random") with a weight of 50 g/m², a thickness of 56 micron and a density of 89 g/dm³.

The band 4 is formed from 35 g/m² siliconized polyester

The two bands 3 and 4 converge towards two counter-rotating pressing rollers 7 and 8 positioned immediately beneath the doctor blade 2. A composite band 9 formed by the combination of the two bands 3 and 4 with a layer of the aforedescribed gel therebetween leaves said rollers, the band 9 being rested on a moving belt which transports it to a machine of known type (not illustrated) which cuts and punches the band to give, for example, pads or patches 10 in the form shown in Figure 2 and which enables the patch itself (obviously after removal of the protective polyester film) to be applied under the nostrils so as to enable the balsamic action of the vapours which develop from the gel layer applied to the patch, the refreshing action due to the evaporation of water and the skin repairing action due to the active components in the gel. In known manner the individual patches 10 can be inserted and preserved in airtight wrappers formed from several combined layers of different materials, for example polyethylene/aluminium/polyethylene.

### EXAMPLE 2

### Preparation of a decongestant nasal patch

28 kg of demineralised water, 0.120 kg of parabens (preservatives), 4.4 kg of polyvinyl alcohol and 0.5 kg of carboxymethylcellulose are fed into a mixer heated to 70°C to obtain a uniform mass (Phase A) which is cooled to ambient temperature and then poured into a kneader into which a Phase C, formed from 0.180 kg of monopropylene glycol, 2.0 kg of carboxymethyl betaglucan (having cicatrising action), 2.5 kg of eucalyptus, clove and black pepper essential oils and 18 kg of sodium alginate, is added while cold (over a period of about 15 to 20 minutes). The mixture is agitated for 15-20 minutes in both directions and then a Phase B, prepared separately by mixing 1.8 kg of demineralized water with 0.5 kg of sodium tetraborate in a steel vessel at a temperature of 20°-30°C until complete dissolution, is slowly added as a thin stream over a period of 20-30 minutes, to give a gel whose viscosity can be increased by increasing the amount of Phase B or decreased by increasing the amount of Phase A.

Proceeding as already described with reference to Figure 1, the gel is spread onto a band of non-woven fabric 3, formed from viscose fibres (50%) and polypropylene fibres (50%), constituting a "random" web structure with a weight of 54 g/m², a thickness of 62 microns and a density of 87 g/dm³.

The protective band 4 consists of a 75 g/m² siliconized polyester sheet.

The composite band 9 leaving the rollers 7, 8 is transported to a machine (not shown) where it is cut and punched taking the form, for example, of the patch 10 shown in Figure 2.

As with the case described in Example 1, the patch thus obtained ensures a refreshing, balsamic action (due to the vapours which evolve from the gel) and skin repairing action (due mainly to carboxymethyl betaglucan).

### EXAMPLE 3

### Preparation of a decongestant nasal patch in which the balsamic action is produced by essential oils subsequently imbibed into the fabric

28 kg of demineralised water, 0.120 kg of parabens (preservatives), 4.4 kg of polyvinyl alcohol and 0.5 kg of carboxymethylcellulose are fed into a mixer heated to 70°C to obtain a uniform mass (Phase A) which is cooled to ambient temperature and then poured into a kneader into which a Phase C formed from 0.180 kg of monopropylene glycol, 2.0 kg of carboxymethyl betaglucan (having cicatrising action), and 18 kg of sodium alginate is added cold (over a period of about 15-20 minutes). The mixture is agitated for 15-20 minutes in both directions and then a Phase B, prepared separately by mixing 1.8 kg of demineralized water with 0.5 kg of sodium tetraborate in a steel vessel at a temperature of 20°-30°C until complete dissolution, is slowly added as a thin stream over a period of 20-30 minutes, to give a gel whose viscosity can be increased by increasing the amount of Phase B or decreased by increasing the amount of Phase A.

Proceeding as already described with reference to Figure 1, the gel is spread onto a band of non-woven fabric coupled to a 35 microns microperforated polythene film 3, formed from viscose fibres (50%) and polypropylene fibres (50%), constituting a "random" web structure with a weight of 150 g/m², a thickness of 200 micron and a density of 87 g/dm³.

The protective band 4 consists of a 75 g/m² siliconized polyester sheet.

The composite band 9 leaving the rollers 7, 8 is transported to a machine (not shown) where it is cut and punched, for example, to assume the form of the patch 10 of Figure 2 and imbibed using two nozzles that spray a mixture composed of Eucalyptus, clove and black pepper essential oils onto the fabric, in a quantity of 200 milligrams per patch.

As with the case described in Example 1, the patch thus obtained ensures a refreshing, balsamic action due to the vapours which are released from the non-woven fabric imbibed with oils, and a skin repairing action (due mainly to carboxymethyl betaglucan). In this instance, said patch ensures there is no contact between essential oils and the skin in cases where such a circumstance is necessary due to irritation problems. Moreover, the coupled plastic film does not allow essential oils to migrate towards the gel.

### EXAMPLE 4

### Preparation of a decongestant nasal patch

A liquid Phase A comprising 18.01 kg of water, 1.05 kg of isopropyl myristate, 0.7 kg of glycerol, 0.18 kg of titanium dioxide, 8.75 kg of carboxymethylcellulose, 1.75 kg of kaolin, 0.7 kg of tartaric acid; 0.7 kg of polysorbate 80, 1.05 kg of sorbitan sesquioleate, 0.7 kg of sodium polyacrylate, 0.35 kg of dihydroxyaluminium aminoacetate, 0.35 kg of propyl parahydroxybenzoate, 0.7 kg of 1,3-butylene glycol and 0.02 kg of phenol methylpropyl paraben is prepared in a mixer heated to 70°C, cooled to ambient temperature and mixing continued for 30 minutes in a kneader into which 0.7 kg of mint essence, 0.35 kg of camphor, 0.35 kg of menthol, 0.35 kg of white thyme and 0.35 kg of liquorice are added and mixing continued for 20 minutes.

Then, again at ambient temperature, 0.35 kg of Hypericum perforatum, 0.35 kg of Calendula and 0.35 kg of Bisabolol are added, while continuing to mix for 20 minutes.

A gel is hence obtained which, in the manner described with reference to Figure 1, is spread (by means of the doctor blade 2) onto a 35 g/m² siliconized polyester protective band. Fixing a distance of 50 micron between the opposing surfaces of the rollers 7 and 8, after leaving said rollers 7, 8 the polyester band with the gel spread thereon is passed through a 1.20 Mev power beta ray chamber for about 30 seconds to promote the polymerisation of the polymerizable components of the gel. A viscose (50%) and polyester (50%) non-woven fabric with a "random" web structure with a weight of 54 g/m², a thickness of 62 microns and a density of 87g/dm³ is then coupled (by means of counter-rotating rollers similar to the rollers 7,8) to that face of the polyester band on which the gel layer is present.

The composite band obtained in this manner is transported by a moving belt to a machine where the band is cut and punched into patches 10 with the profile shown in Figure 2, which are then enclosed, preserved and sealed in wrappers of known type (formed from layers of polyethylene/aluminium/polyethylene).

The patch described can be applied on the upper lip and under the nostrils, to enable the already stated refreshing balsamic action of the vapours, and the skin repairing action.

### EXAMPLE 5

### Preparation of a pad for ocular gel

A liquid Phase A consisting of 32 kg of demineralised water, 0.120 kg of parabens (preserving agents), 4.8 kg of polyvinyl alcohol and 0.150 kg of chlorhexidine is prepared in a mixer heated to 70°C; the mass obtained is cooled and poured into a kneader into which 0.180 kg of monopropylene glycol, 0.140 kg of 70% sorbitol, 0.5 kg of betaglycyrrhetic acid, 0.8 kg of pineapple extract, 0.8 kg of Echinacea extract and 18 kg of sodium alginate are added, stirring in both directions for 15-20 minutes.

A solution of sodium tetraborate (0.200 kg) and demineralised water (0.8 kg) is prepared separately in a steel mixer heated to 25-30°C, to give a Phase B which is slowly added as a thin stream to the aforesaid kneader in which the mass is maintained in movement for a period of 20-30 minutes, giving rise to the formation of a gel whose fluidity can be increased by increasing the quantity of the Phase B, or decreased by increasing the quantity of the Phase A.

Using the system outlined in Figure 1, said gel is spread, by means of a doctor blade 2 and two counter-rotating blades 7, 8, between a band of 50 g/m² pure viscose fabric and a protective band of 70 g/m² siliconized PET and is then cut, punched and wrapped.

In this manner pads 11 are obtained having the elliptical shape shown in plan view in Figure 3, a plurality of perforations 12 being provided along the periphery of the pad itself, which at its centre is also provided with a long longitudinal incision 13.

The shape of the pad allows it to be applied over an eye so as to lightly compress the eyelids, allowing any discharge to drain from the perforations 12 and incision 13.

If necessary, a conventional plaster can be placed over the pad to retain the pad firmly on the eye.

### EXAMPLE 6

### Preparation of a pad for ocular gel

33 kg of demineralised water, 0.150 kg of parabens (antioxidants and preservatives), 5.3 kg of polyvinyl alcohol, and 0.8 kg of carboxymethylcellulose are fed into a mixer heated to 70°C and stirred until dissolution is complete. The mixture is cooled and poured into a kneader into which 0.140 kg of 70% sorbitol, 0.8 kg of betaglycyrrhetic acid, 0.3 kg of pineapple extract, 0.3 kg of Echinacea extract and 12 kg of sodium alginate are added and is mixed in both directions at ambient temperature for about 20-30 minutes, after which a Phase B, prepared separately by mixing 1.2 kg of demineralised water with 0.150 kg of sodium tetraborate at 25-30°C in a steel vessel until dissolution is complete, is slowly added as a thin stream. A gel is obtained whose fluidity can be increased by increasing the quantity of Phase A or decreased by increasing the quantity of Phase B.

The gel can be spread in the manner already described onto a 50 g/m² non-woven fabric pad of pure viscose protected by a 70 g/m² sheet of siliconized PET, which can be then punched to assume the form of Figure 3 and then be applied over an eye to develop a localized cooling, anti-edemic and healing effect, in exactly the same manner as described in Example 5.

### EXAMPLE 7

### Preparation of a pad for ocular gel

A liquid Phase A formed of

| | |
|---|---|
| water | 17.85 |
| gelatin | 2.8 |
| polyvinylpyrrolidone | 4.55 |
| sorbitol 70 | 0.88 |
| Kaolin | 0.88 |
| titanium dioxide | 0.18 |
| monopropylene glycol | 1.05 |
| methylparahydroxybenzoate | 0.35 |
| propylparahydroxybenzoate | 0.35 |
| disodium edetate | 0.18 |
| tartaric acid | 0.18 |
| dihydroxyaluminium aminoacetate | 0.18 |
| carboxymethylcellulose | 4.90 |
| sodium polyacrylate | 0.70 |

the values being expressed in kg, is prepared in a mixer heated to 70°C.

The homogenous mass thus obtained is cooled to ambient temperature, while continuing to mix for 30 minutes, then 0.05 kg of mint and 0.10 kg of liquorice are added and mixed for a further 20 minutes and finally 0.7 kg of glycolic extract of pineapple, 0.7 kg of glycolic extract of Echinacea and 0.35 kg of betaglycyrrhetic acid are added, continuing to mix for a further 20 minutes.

In this manner a gel is obtained which is spread (by means of a doctor blade and two counter-rotating rollers spaced 50 microns apart) onto a 35 g/m² band of siliconized polyester. The polyester band with the gel spread thereon is passed through a chamber where it is subjected to radiation by UVA rays of about 220 nanometers for about 30 seconds to promote polymerisation, and is then coupled to a viscose (50%) and polyester (50%) non-woven fabric band, of "random" web structure, with 54 g/m² weight, 62 microns thickness and 87 g/dm³ density.

By punching, pads are obtained similar to that shown in Figure 3, which can be applied over the eyes to provide an effective refreshing balsamic action by the vapours and a skin repairing action.

## Claims

1. Pad for application to the human skin, for use in developping a decongestant, cosmetic and/or pharmaceutical action **characterised by** consisting of a flexible porous support (3), on at least one surface of which is applied a layer of gel (1) consisting of an intimate mixture comprising between 50% and 77% of water, between 6.5% and 44% of polyvinyl alcohol, between 0% and 10% of a substance of plant origin comprising essential oils and aromatic extracts; and between 0% and 10% of at least one dermatologically compatible component chosen from the group comprising soothing, skin repairing, cicatrising, anti-inflammatory, antiseptic and bactericidal substances; said gel (1) further comprising between 0.01% and 5.2% of an alkaline or alkaline earth metal tetraborate, the percentages being by weight.

2. Pad as claimed in claim 1, **characterised in that** said support (3) consists of a non-woven fabric made of fibres chosen from the group consisting of viscose, polyester and polyethylene fibres, mixed polyester/viscose fibres, cotton and linen, said fabric support having a thickness between 15 and 200 microns, a density from 65 to 145 g/dm³ and a weight between 15 and 150 g/m².

3. Pad as claimed in claims 1 or 2, **characterised in that** said gel (1) comprises between 0.001% and 5.2% of at least one dermatologically compatible auxiliary component chosen from the group-consisting of solvents, wetting agents, preservatives, emulsifiers, stabilizers, solubilizers, surfactants, colorants, the percentages being by weight.

4. Pad as claimed in claims from 1 to 3, **characterised in that** said flexible porous support (3) is impregnated with said substance of plant origin, present in a quantity between 0% and 10% by weight on the total weight of the gel (1) applied to the support.

## Patentansprüche

1. Pad zur Applikation auf die humane Haut zur Verwendung bei der Entwicklung einer abschwellenden, kosmetischen und/oder pharmazeutischen Wirkung, **dadurch gekennzeichnet, dass** es aus einem flexiblen porösen Träger (3) besteht, bei dem auf wenigstens eine Oberfläche eine Schicht aus Gel (1) aufgetragen ist, bestehend aus einem innigen Gemisch, umfassend zwischen 50% und 77% Wasser, zwischen 6,5% und 44% Polyvinylalkohol, zwischen 0% und 10% einer Substanz pflanzlicher Herkunft, die essentielle Öle und aromatische Extrakte umfasst; und zwischen 0% und 10% wenigstens einer dermatologisch kompatiblen Komponente, ausgewählt aus der Gruppe, umfassend lindernde, hautreparierende, narbenbildende, antiinflammatorische, antiseptische und bakterizide Substanzen, wobei das Gel (1) außerdem zwischen 0,01% und 5,2% eines Alkalimetall- oder Erdalkalimetalltetraborats umfasst, wobei die Prozentangaben Gewichtsprozent sind.

2. Pad, wie es in Anspruch 1 beansprucht ist, **dadurch gekennzeichnet, dass** der Träger (3) aus einem Non-Woven-Gewebe besteht, das aus Fasern hergestellt ist, die ausgewählt sind aus der Gruppe, bestehend aus Viskose-, Polyester- und Polyethylenfasern, Polyester/Viskose-Mischfasern, Baumwolle und Leinen, wobei der Gewebeträger eine Dicke von zwischen 15 und 200 Mikrometern, eine Dichte von 65 bis 145 g/dm³ und ein Gewicht zwischen 15 und 150 g/m² hat.

3. Pad, wie es in Anspruch 1 oder 2 beansprucht ist, **dadurch gekennzeichnet, dass** das Gel (1) zwischen 0,001% und 5,2% wenigstens einer dermatologisch kompatiblen Hilfskomponente umfasst, die ausgewählt ist aus der Gruppe, bestehend aus Lösungsmitteln, Netzmitteln, Konservierungsmitteln, Emulgatoren, Stabilisatoren, Solubilisierungsmitteln, Surfactants, Färbemitteln, wobei die Prozentangaben Gewichtsprozent sind.

4. Pad, wie es in den Ansprüchen 1 bis 3 beansprucht ist, **dadurch gekennzeichnet, dass** der flexible poröse Träger (3) mit der Substanz pflanzlicher Herkunft imprägniert ist, wobei diese in einer Menge zwischen 0 Gew.-% und 10 Gew.-%, bezogen auf das Gesamtgewicht des auf den Träger aufgetragenen Gels (1), vorliegt.

## Revendications

1. Compresse à appliquer sur la peau humaine, à utiliser dans le développement d'une action décongestive, cosmétique et/ou pharmaceutique, **caractérisée en ce qu'**elle est constituée d'un support poreux flexible (3) sur au moins une surface duquel est appliquée une couche de gel (1) consistant en un mélange intime comprenant entre 50 % et 77 % d'eau, entre 6,5 % et 44 % d'un alcool de polyvinyle, entre 0 % et 10 % d'une substance d'origine végétale comprenant des huiles essentielles et des extraits aromatiques ; et entre 0 % et 10 % d'au moins un composant dermatologiquement compatible choisi dans le groupe comprenant un calmant, un agent de réparation cutanée, un cicatrisant, un anti-inflammatoire, un antiseptique et des substances bactéricides, ledit gel (1) comprenant en outre entre 0,01 % et 5,2 % d'un tétraborate de métal alcalin ou de métal alcalino-terreux, les pourcentages étant en poids.

2. Compresse selon la revendication 1, **caractérisée en ce que** ledit support (3) consiste en un tissu non tissé constitué de fibres choisies dans le groupe comprenant des fibres de viscose, de polyester et de polyéthylène, des fibres mixtes de polyester/viscose, de coton et de lin, ledit support de tissu ayant une épaisseur entre 15 et 200 microns, une densité de 65 à 145 g/dm³ et un poids entre 15 et 150 g/m².

3. Compresse selon les revendications 1 ou 2, **caractérisée en ce que** ledit gel (1) comprend entre 0,001 % et 5,2 % d'au moins un composant auxiliaire dermatologiquement compatible choisi dans le groupe comprenant les solvants, les agents mouillants, les conservateurs, les émulsifiants, les stabilisants, les solubilisants, les tensioactifs, les colorants, les pourcentages étant en poids.

4. Compresse selon les revendications 1 à 3, **caractérisée en ce que** ledit support poreux flexible (3) est imprégné de ladite substance d'origine végétale, présente en une quantité entre 0 % et 10 % en poids du poids total du gel (1) appliqué sur le support.
